# EUROPEAN PATENT SPECIFICATION

(11) **EP 2 565 175 B1**
(45) Date of publication and mention of the grant of the patent: **21.04.2021**
(21) Application number: 11179515.9
(22) Date of filing: 31.08.2011
(51) Int. Cl.: C07C 29/60

(54) **Catalytic process for the production of 1,2-propanediol from crude glycerol stream**
Katalysatorverfahren zur Herstellung von 1,2-propandiol aus einem Rohglycerolstrom
Procédé catalytique pour la production de 1,2-propanediol à partir de flux de glycérol brut

(43) Date of publication of application: 06.03.2013
(73) Proprietor: Aristotle University of Thessaloniki, 54636 Thessaloniki (GR); Lemonidou, Angeliki, 54124 Thessaloniki (GR); Vasileiadou, Efterpi, 54124 Thessaloniki (GR)
(72) Inventor: Lemonidou, Angeliki, 54124 Thessaloniki (GR); Vasileiadou, Efterpi, 54124 Thessaloniki (GR)
(74) Representative: Petsis, Christos

(56) References cited:
- WO-A1-2008/077205
- US-A1- 2008 025 903

## Description

### Field of the invention

The present invention relates to a catalytic process for hydrogenating glycerol for the production of lower alcohols, particularly to the formation of 1,2-propanediol, also known as propylene glycol, using glycerol by-product as a raw material without the need of hydrogen gas addition for the hydrogenation reaction to occur.

### Background of the invention

Nowadays, the uncertain supply of fossil fuels and global warming problems resulted in the increase of renewable fuel production, especially biodiesel from transesterification of vegetable oils and fats. The overproduction of crude glycerol is a direct result of the increase in biodiesel production, as it is generated as a side-product of the process. The latter created volatile pricing throughout the industry, while the demand for glycerol in general has remained stable. Without refinement, this impure form of glycerol must either be disposed in a certain amount of time or sold off to market for a minimal value due to the water, methanol, and salt content. Alternative uses of glycerol has emerged great interest and especially the transformation to value added chemicals that are now currently produced from petroleum resources. Hydrogenation of glycerol to 1,2-propanediol is one of the most attractive methods to utilize glycerol and offer additional value to a biodiesel plant.

1,2-propanediol, a raw material for antifreeze, personal care products and polymeric resins, is currently produced from petrochemical sources and it is predicted that in the future its production may decrease due to limited adequacy in oil reserves.

During the biodiesel production process, the stream of the crude glycerol mainly consists of a mixture of glycerol, methanol and water. Nowadays, methanol is separated and recycled to the reactor.

Hydrogen as a reactant and a suitable catalyst are needed to activate dehydration and hydrogenation reactions of glycerol. Hydrogen use is costly and this has resulted in an increase of the cost of final product and limits this important reaction to laboratory scale.

### Prior Art

Earlier work published in the open literature reports the progress that has been achieved over the past years for hydrogenation or hydrogenolysis processes of glycerol conversion to propylene glycol. The hydrogenation of glycerol, in general takes place in the presence of metal catalysts under 180-240°C in the case of liquid phase reaction and 5-100 bar hydrogen atmosphere. Two categories of metal catalysts have been proved to effectively catalyze this reaction. Noble metal catalysts with ruthenium-based as the most representative materials and other transition metal catalysts with copper-based the most efficient catalysts.

C. Montassier et al in J. Mol. Catal. 70 (1991) 99 described polyol conversions (such as xylose, glycerol, xylitol and glycitol) in the presence of ruthenium and sulfur modified ruthenium catalysts under 190-230°C and 4-6 MPa hydrogen pressure in the case of glycerol feedstock.

Dasari et al at in Appl. Catal. A Gen. 281 (2005) 225 described a low-pressure hydrogenolysis process of glycerol to propylene glycol using commercial copper-chromite catalyst under 200psi hydrogen pressure and 200°C. A pathway of the reaction was also proposed by isolating acetol intermediate.

Maris and Davis J. Catal. 249 (2007) 328 disclosed hydrogenation of glycerol over commercial noble metal, ruthenium and platinum catalysts supported on carbon at 200°C and 40 bar hydrogen pressure using very diluted aqueous solution feedstocks of 1wt%glycerol. The best results were obtained in the presence of base additives, such as CaO and NaOH.

Wang and Liu Catal. Let. 117 (2007) 62 reported the performance of synthesized Cu/ZnO catalysts with 20wt% glycerol aqueous solution under 180 to 240°C and 42 bar hydrogen pressure. The article states that the combination of copper with zinc oxide provides the bifunctional sites needed for the hydrogenolysis reaction of glycerol.

More recently, R.B. Mane et al Catal. Lett. 135 (2010), 141 synthesized a non-chromium Cu:Al catalytic material which showed better performance than a bulk Cu-Cr catalyst when tested in both water and isopropanol solutions of 20wt% glycerol at 220°C and 70 bar hydrogen pressure.

Vasiliadou and Lemonidou reported the formation of 1,2-propanediol from glycerol over synthesized Ru-based catalysts (Appl. Catal. B Env. 92 (2009) 90 and Org. Process Res. Dev. (2011) DOI: 10.1021/op2000173) and Cu-based catalysts (Appl. Catal. A Gen. 396 (2011) 177). The experiments were carried out at 240°C and under 80 bar hydrogen atmosphere. The results of these studies demonstrate the superior performance of copper based catalysts in terms of 1,2-propanediol selectivity and show the positive effect of small copper particle size on activity expressed as turnover frequency.

Wawrzetz et al. in J. Catal. 269 (2) (2010) 411 published glycerol reactions in aqueous solutions using a Pt/Al₂O₃ catalyst and reported the production of 1,2-propanediol under inert atmosphere at 15% yield under 225°C and 29 bar nitrogen system pressure.

More recently, D. Roy et al in Catal. Tod. 156 (1-2) (2010) 31 reported aqueous phase glycerol hydrogenolysis without external hydrogen addition using an admixture of 5wt%Ru/Al2O3 and 5wt%Pt/Al2O3. At 220°C and 14 bar nitrogen inert atmosphere after 6h the 1,2-propanediol yield was 23,6% with 50.2 conversion and 47.2 selectivity.

The state-of-the-art also contemplates some patent documents related to processes for hydrogenating or hydrogenolyzing glycerol.

U.S. Patent 5.616.817 refers to a process for the preparation of 1,2-propanediol by catalytic hydrogenation of glycerol at elevated temperature, preferably from 200 to 250°C, and elevated pressure, preferably from 200 to 325 bar. The process described comprises using glycerol having a water content of up to 20% by weight and a catalyst comprising the metals cobalt, copper, manganese and molybdenum in amounts of, based on the total weight of the catalyst, from 40 to 70% by weight of cobalt, from 10 to 20% by weight of copper, from 0 to 10% by weight of manganese and from 0 to 10% by weight of molybdenum, where this catalytically active material may additionally contain inorganic polyacids and/or heteropolyacids in an amount of up to 10% by weight, based on the total weight of the catalyst.

Another method which is included in US 5.214.219 describes a process of hydrogenating glycerol in liquid phase over a copper/zinc catalysts under a temperature above 200°C and a hydrogen pressure of 5-20 MPa. The feedstock can be an either aqueous or alcoholic solution of glycerol or in general impure glycerol from saponification or transesterification of fats treated with an ion-exchange resin. After 2h at 270°C and hydrogen pressure of 10 MPa, a 30 wt% aqueous glycerol solution was almost fully converted with 1,2-propanediol selectivity reaching 84%.

US 5,276,181 refers to a method for obtaining 1,2-propanediol at 75% yield using severe process conditions. Glycerol conversion reaches 100% by using 30 wt% aqueous solution at 240°C, 13 MPa, 2h over a RuS/C heterogeneous catalyst and NaOH addition in the reaction mixture.

In EP 1440 046 an 25 wt% glycerol aqueous solution under high pressure hydrogen atmosphere of 12,4 MPa and 230°C and 4h reaction time is converted to 1,2-propanediol. The catalytic system used is a multimetallic heterogeneous ReNi catalyst with NaOH addition as a promoter additive. 1,2-propanediol yielded 54% at 61% conversion and 88% selectivity.

In US 0022807/2010 D'Hont et al describe a process for a single step production of oxygenated hydrocarbons, and more preferably 1,2-propanediol from glycerol in aqueous medium. The single step refers to a process where the conversion runs in absence of added hydrogen and is carried out under inert atmosphere. The heterogeneous catalyst used is a noble metal catalyst, preferably platinum, supported on faujasite-type zeolite carrier. Using a 20 wt% glycerol in water and 4 wt% of the Platinum-based catalyst at 230°C after 17h under nitrogen atmosphere a 53% 1,2-propanediol yield is achieved with 90% conversion and 59% selectivity.

In the case of inert atmosphere processes studied until now (Wawrzetz et al. in J. Catal. 269 (2) (2010) 411, D. Roy et al in Catal. Tod. 156 (1-2) (2010) 31, US 0022807/2010 D'Hont et al) the reaction sequence involves the use of a part of glycerol feedstock to produce the hydrogen needed for hydrogenation of the remaining glycerol. In addition, the requirement of a noble metal catalytic system for the above methods is considered essential.

In addition, in the known art, US 5,426,249 and US 2010/0240493 A1, thus describe essentially multistep processes contacting the reacting substrates in different reactor cascades, while the present invention aims at providing the ability of using one reactor set up.

However, yet existing single step processes, such as described in EP 1440446, US 5,616,817, US 5,276,181 and US 5,214,219 require high hydrogen pressures in order to obtain high 1,2-propanediol yields. Montassier et al (J. Mol. Catal. 70 (1) (1991) p. 99-110 also describe a method of hydrogenation glycerol substrate by applying high hydrogen pressures of at least 3 MPa. Furthermore, there are some examples (Dasari et al Appl. Catal. A Gen 281 (2005) p. 225-231 and Suppes et al WO 2005/095536 A2) operate under milder conditions, such as 200°C and 1,4 MPa of hydrogen. In all the above methods of hydrogenating glycerol, in order to obtain high yields of lower alcohols and especially 1,2-propanediol, the addition of hydrogen gas is an essential parameter.

### Object of the invention

It is an object of the present invention to effectively utilize the crude glycerol stream avoiding separation steps after transesterification. It is also an object of the present invention to run the hydrogenation reaction of glycerol under inert system pressure excluding the external addition of hydrogen gas. It is further an object of the present invention to proceed the reaction in liquid phase eliminating the need of heat of vaporization of oxygenate feedstocks. It is finally an object of the present invention to maximize the yield of 1,2-propanediol product preparation.

The invention generally describes a new method referring to one step conversion of crude glycerol stream to 1,2-propanediol without the need of external addition of hydrogen.

Besides, the present invention proposes the direct use of the crude glycerol stream into a hydrogenation reactor, where glycerol is converted to propylene glycol in one step.

There is thus proposed a catalytic process for hydrogenating glycerol for the production of lower alcohols, which is remarkable according to the invention, in that the process of glycerol conversion is a one-step catalytic process which is independent or does not require the external addition of hydrogen gas, comprising subjecting a water, methanol and glycerol reaction mixture to heating under inert nitrogen system pressure over a metal heterogeneous catalyst and to set the reaction mixture in amounts sufficient to induce or to control catalytic hydrogenation.

The hydrogen required to react is produced in situ by the reaction of methanol with water and consumed by glycerol to form 1,2-propanediol.

Although various catalytic processes for the one step conversion of glycerol to lower alcohols such as 1,2-propanediol have been developed, none of them show the advantages of this invention. The present one step process does not require external hydrogen addition to hydrogenate glycerol, takes advantage of the methanol contamination and water, which are already components of the crude glycerol stream after transesterification, to produce the needed hydrogen, the needed hydrogen mainly forms from methanol and water and limits the "sacrifice" of glycerol substrate, requires no use of homogeneous additives, uses the same conditions, catalyst and reactor, proceeds in liquid phase and therefore in milder conditions and realizing the use of crude glycerol stream avoiding separation steps.

An object of the present invention is the production of 1,2-propanediol from glycerol stream that also contains methanol and water, obtainable from transesterification processes of fats and oils, in a one-step manner with mainly bio-propanols and bio-ethylene glycol as co-products.

### Summary of the invention

According to the present invention, there is proposed a catalytic process as defined in claim 1, i.e. for hydrogenating glycerol for the production of lower alcohols, which is remarkable in that
- first, a crude glycerol stream containing methanol mainly consisting of a mixture of glycerol, methanol and water is obtained in a process for producing biodiesel from transesterification of vegetable oils and fats,
- followed by a one-step catalytic process of glycerol conversion
wherein the crude glycerol stream is used avoiding separation steps, wherein said water, methanol and glycerol reaction mixture is subjected to heating under inert nitrogen system pressure at temperatures of 220-260°C and a system initial pressure of 15-50 bar, wherein the one-step catalytic process is independent of the addition of hydrogen gas or does not require the external addition of hydrogen gas,
and wherein in the one-step catalytic process the reaction is carried out over a supported platinum metal catalyst or a bulk catalyst comprising copper.

There is thus provided a one step process for the production of oxygenated hydrocarbons, preferably 1,2-propanediol, from glycerol. The method comprises the transformation of methanol and water to hydrogen and carbon dioxide and the consumption of the produced hydrogen for the hydrogenation of glycerol to the selective production of 1,2-propanediol.

The liquid phase nature of the process minimizes undesirable decomposition reactions and carbon monoxide formation, as the above typically encountered when carbohydrates are heated to elevated temperatures.

A suitable catalyst comprises platinum deposited on a carrier. The carrier may be a solid oxide, such as silica and alumina.

In accordance with the present invention, it is broadly referred to a one-step process for the production of lower alcohols, particularly three-carbon chain alcohols, such as 1-propanol, 2-propanol and 1,2-propanediol and ethylene glycol from glycerol stream by contacting the above stream with the catalyst according to the invention.

Suitable metal catalysts consist of mainly two categories of catalytic materials :
A i) supported metal Pt and ii) a porous support, preferably an oxidic support such as alumina and silica,
B: bulk catalysts comprising a first component, metal Cu and the combination of two other oxidic components, such as zinc oxide and aluminum oxide.

In a preferred embodiment according to the present invention, there is provided a process for the preparation of a mixture comprising mainly 1,2-propanediol from glycerol which includes:
(a) reacting a reaction mixture of glycerol, methanol, water and a metal catalyst
(b) the reaction proceeds in liquid phase under inert atmosphere at temperature between 220-260°C and pressure from 15-50 bar and
(c) the hydrogen gas needed for the hydrogenation of glycerol is in situ produced by methanol and water components.

In one aspect according to the present invention, the method provides a process of converting glycerol stream obtained in the transesterification process, into a mixture of valuable products, like 1,2-propanediol, 1-propanol, 2-propanol and ethylene glycol.

In a specific aspect according to the present invention, a one-step process converts a glycerol stream to 1,2-propanediol by contacting the substrate with a catalyst consisting of
i) supported metal Pt and ii) a porous support, preferably an oxidic support such as alumina or a bulk catalyst comprising a first component metal Cu and the combination of two other oxidic components, such as zinc oxide and aluminum oxide.

According to the present invention, a suitable metal catalyst is Pt.

In a particular embodiment, the Pt-based catalyst comprises a metal content ranging from 1-8 wt% and more preferably 4-6 wt%.

In a preferred embodiment, the supported catalyst consists of a porous oxidic support, such as silicon oxide and aluminum oxide.

In a particularly preferred embodiment, the suitable catalyst could also be a bulk catalyst with the first metal component being Cu. The bulk copper catalyst comprises a first component containing at least one of copper or copper oxide and a second component containing zinc oxide. It is also preferable that the catalyst contains a third component other than copper, copper oxide, or zinc oxide, more preferably a high surface area oxidic component and even more preferably aluminum oxide as the third component.

According to a preferred embodiment of the invention, the catalyst preferably satisfies a weight ratio of the first component to the second component to be in a range of 20/80 to 80/20 and more preferably in a range of from 35/65 to 65/35. The third component content should be 25 wt% or less.

In a more preferred embodiment according to the present invention, said metal catalyst is prepared by impregnation, co-precipitation or gel-co precipitation of oxalate precursor methods as widely described in the known art.

In a particularly preferred embodiment according to the present invention, a one-step process for the production of lower alcohols, such as 1,2-propanediol, is carried out using a glycerol containing feedstock with a concentration range of 1-50 wt% glycerol in water and methanol media. Concentration of glycerol should be between preferably 5-30 wt% and most preferably between 5-15 wt%. Methanol concentration should be between preferably 1-30 wt% and most preferably between 2-15 wt%. The water content should be between 40-94 wt% and most preferably between 70-93 wt%.

The process of the present invention converts glycerol to lower alcohols such as 1-propanol, 2-propanol and 1,2-propanediol, with a combined selectivity for lower alcohols of 47,3%, a selectivity for 1,2-propanediol of 39,2% and yield of 34,8%, at glycerol conversion of 85% or higher.

Further characteristics of the present invention relate to the process parameters and more specifically to the temperature and system initial pressure. The temperature used in the reaction ranges from 220-260°C. The initial pressure used in the reaction ranges from 15-50 bar.

In still another aspect of the invention, the hydrogenation reaction of glycerol for the production of 1,2-propanediol is realized without the need of external hydrogen addition and is carried out in the presence of inert nitrogen atmosphere.

Further scope of applicability of the present invention will become apparent from the detailed description given hereinafter. However, it should be understood that the detailed description and specific examples, while indicating preferred embodiments of the invention are exemplary and explanatory only.

The present invention concerns a catalytic process for the production of lower alcohols from glycerol containing streams, which is remarkable in that the process of glycerol conversion is a one-step catalytic process which is independent of the addition of hydrogen gas or does not require addition of hydrogen to proceed comprising subjecting water/methanol/glycerol reaction mixture to heating in the presence of a heterogeneous catalyst comprising a supported metal or bulk catalyst and to set the reaction mixture in parameters sufficient to induce or to control catalytic hydrogenation of glycerol. In this process glycerol can be converted to 1,2-propanediol under inert nitrogen atmosphere over a metal catalyst.

In this process glycerol can be converted with the reaction mixture under inert nitrogen atmosphere. A required catalyst for this method should effectively catalyze the in-situ formation of hydrogen gas from methanol and water and its subsequent use in the hydrogenation of glycerol to 1,2-propanediol production. The catalyst used in this process is A (i) a heterogeneous supported catalyst from the group consisting of metal Pt and (ii) a porous support, such as an oxidic support such as alumina, silica or B a bulk catalyst comprising a first component a metal Cu and the combination of two other oxidic components such as zinc oxide and aluminum oxide.

Such catalyst can be prepared by impregnation, co-precipitation or gel-co precipitation of oxalate precursors methods. In a particular embodiment the amount of platinum should be ranging from 1-8 wt% and more preferably 4-6 wt%. The suitable metal catalyst is Pt. The bulk catalyst comprising copper comprises a first component containing at least one of copper or copper oxide and a second component containing zinc oxide. It is also preferable that the catalyst contains a third component other than copper, copper oxide or zinc oxide more preferably a high surface area oxidic component and even more preferably aluminum oxide as the third component.

The reaction temperature for the catalytic process ranges from 220-260°C. The initial pressure used in the reaction preferably ranges from 15-50 bar inert nitrogen atmosphere.

The abovementioned conditions and materials are used in such process of the present invention.

### Detailed description

The term "one step process" refers to a sequence of chemical transformation occurring in a single reactor.

The term "oxidic support" as used herein refers to a solid oxide of type MₙOₙ₊₁ with suitable surface area and mild acidity. It includes but it is not limited to materials like SiO₂-type and Al₂O₃.

There is provided a one step process for the production of oxygenated hydrocarbons, preferably 1,2-propanediol from a crude glycerol stream containing methanol mainly consisting of a mixture of glycerol, methanol and water is obtained in a process for producing biodiesel from transesterification of vegetable oils and fats. The method comprises the transformation of methanol and water tc hydrogen and carbon dioxide and the consumption of the produced hydrogen for the hydrogenation of glycerol to the selective production of 1,2-propanediol.

The liquid phase nature of the process minimizes undesirable decomposition reactions and carbon monoxide formation, as the above typically encountered when carbohydrates are heated to elevated temperatures.

A suitable catalyst comprises platinum deposited on a carrier. The carrier may be a solid oxide, such as silica and alumina.

The Platinum-based catalyst is preferably prepared by wet impregnation method. For impregnation, the desired amount of metal precursor is dissolved in a solvent, usually deionized water. While adding the metal precursor solution to the catalyst carrier, the solid is homogenized. After solvent evaporation by using a rotary evaporator under vacuum, drying at 110°C overnight, the solid is treated in flowing air at temperatures preferably between 350°C and 450°C and reduced in flowing mixture of hydrogen/nitrogen up to temperatures ranging preferably between 200°C to 300°C.

The metal of the preferred catalyst of the above category (Group VII) is platinum. Using water as a solvent during impregnation the preferred metal precursor is dihydrogen hexachloroplatinate (IV). The preferred weight percentage of platinum deposited on the carrier amounts between 1-8 wt% and more preferably 4-6 wt%.

Another suitable catalytic system of the present invention comprises copper. This bulk catalyst consists of a second component containing zinc oxide. It is also preferable that the catalyst contains a third component other than copper, copper oxide or zinc oxide more preferably a high surface area oxidic component and even more preferably aluminum oxide as the third component.

The Copper-based catalyst is preferably prepared by standard well known techniques including but not limited to co-precipitation, gel-co precipitation of oxalate precursor, such as described in article reports [M. Balaraju et al Catal. Lett. (2008) p. 119-124 and X.-R. Zhang et al Catal. Lett. (2005) 102 p. 183-190]. The drying proceeds at 110°C overnight and then the solid is treated in flowing air at temperatures preferably between 350°C and 450°C and reduced in flowing mixture of hydrogen/nitrogen up to temperatures ranging preferably between 300°C and 450°C. The metal precursors preferred are nitrate salts of each component, while the composition preferably satisfies a weight ratio of the first component to the second component to be in a range of 20/80 to 80/20 and more preferably in a range of from 35/65 to 65/35. The third component content should be 25 wt% or less. The metal precursors preferred are the nitrate salts of each metal component.

Typically, the reactions are carried out in a stainless steel batch reactor of 450 ml connected to inlet and outlet of gases. The reactor is equipped with an electronic temperature controller, a mechanical stirrer and a tube for the sampling of the liquid phase.

However, no limitations are placed or implied on whether reactions are carried out continuously or in (fed)-batch type reactors. The process according to the present invention is not limited to a certain set up of equipment, as long as it is taking care that the feed forms a single phase before entering the catalyst.

The reaction is carried out at a temperature ranging from 220-260°C. The initial pressure used in the reaction ranges from 15-50 bar inert nitrogen atmosphere.

It is preferred that the concentration ranges between 1-50 wt% glycerol in water and methanol media. Preferably concentration of glycerol should be between 5-30 wt% and most preferably between 5-15 wt%. Preferably concentration of methanol should be between 1-30 wt% and most preferably between 2-15 wt%. The water content should be between 40-94 wt% and most preferably between 70-93 wt%.

The optimal reaction time depends on the applied catalyst, amount of catalyst, reactant concentration and temperature and pressure conditions. With suitable setting of the above parameters a substrate conversion of 85% or higher with a combined selectivity for lower alcohols of about 47,3%, a selectivity for 1,2-propanediol of about 39,2% and yield of 34,8%. 1,2-propanediol is the preferred product of this invention.

### Examples

The term conversion (X, %) is the amount of moles of glycerol reacted under the conditions expressed as % of the initial moles of glycerol.

The term selectivity (S, %) of a product is defined as the number of Carbon moles of the specific product divided by the number of Carbon moles of glycerol converted multiplied by 100. The term lower alcohol selectivity includes the selectivity of C3 atom alcohol components such as 1,2-propanediol, 1-&2-propanols.

The term yield (Y, %) is determined as moles of specific product divided by initial glycerol moles multiplied by 100.

### Example 1 according to the invention

### Glycerol Catalytic Conversion According to the invention

The reaction is carried out in a 450 ml stainless steel batch reactor with stirring at 1000 rpm, 7,2 wt% methanol, 11,4 wt% glycerol and water. The weight ratio of catalyst to glycerol was 0,1. The reactor was first purged with nitrogen gas and then pressurized to the desired initial system pressure of 35 bar nitrogen atmosphere. Then reactor was heated to the desired reaction temperature of 250°C and was kept to this temperature for 4h. After cooling to room temperature the liquid and gas phase were offline analyzed. The liquid samples were analyzed using gas chromatography on a DB-Wax (30 m x 0,53 mm x 1,0 µm) column and a flame ionization detector. The initial temperature of 35°C was kept constant for 7minutes and then the temperature increased with 10°C/min at the final temperature of 215°C. The gas samples were also analyzed by gas chromatography employing two columns, Porapak Q and Molecular Sieve-5A in series by-pass configuration and a thermal conductivity detector.

### Catalyst: A: 5wt%Pt/γ-Al₂O₃, B: 5wt%Pt/SiO₂

**Table 1**

| Catalyst | Glycerol conversion, % | Lower alcohol selectivity, % | 1,2-propanediol selectivity, % | 1,2-propanediol yield, % | 1-propanol selectivity, % | 2-propa nol selectivity, % |
|---|---|---|---|---|---|---|
| A | 70,4 | 46,6 | 9,5 | 6,7 | 28,7 | 8,4 |
| B | 58,9 | 61,8 | 36,2 | 21,4 | 12,6 | 13 |

### Example 2 according to the invention

The reaction is carried out as in example 1 but at different temperatures over the catalyst B.

**Table 2**

| Temperature, °C | Glycerol conversion, % | Lower alcohol selectivity, % | 1,2-propanediol selectivity, % | 1,2-propanediol yield, % | 1-propanol selectivity, % | 2-propanol selectivity, % |
|---|---|---|---|---|---|---|
| 225 | 19 | 89,5 | 65,6 | 12,6 | 9,5 | 14,4 |
| 250 | 58,9 | 61,8 | 36,2 | 21,4 | 12,6 | 13 |
| 255 | 60,9 | 60,2 | 33,3 | 20,3 | 13,8 | 13,1 |

### Example 3 according to the invention

The reaction is carried out as in example 1 but at variable reaction times over the catalyst B.

**Table 3**

| Reaction time, h | Glycerol conversion, % | Lower alcohol selectivity, % | 1,2-propanediol selectivity, % | 1,2-propanediol yield, % | 1-propanol selectivity, % | 2-propanol selectivity, % |
|---|---|---|---|---|---|---|
| 4 | 58,9 | 61,8 | 36,2 | 21,4 | 12,6 | 13 |
| 8 | 62,8 | 47,7 | 27,6 | 17,4 | 14,2 | 5,9 |

### Example 4 according to the invention

The reaction is carried out as in example 1 but with different methanol concentration over the catalyst B.

**Table 4**

| Methanol concentration, w/w% | Glycerol conversion, % | Lower alcohol selectivity, % | 1,2-propanediol selectivity, % | 1,2-propanediol yield, % | 1-propanol selectivity, % | 2-propanol selectivity, % |
|---|---|---|---|---|---|---|
| 3,7 | 43,2 | 52,5 | 37,9 | 16,4 | 8,6 | 6 |
| 7,2 | 58,9 | 61,8 | 36,2 | 21,4 | 12,6 | 13 |

### Example 5 according to the invention

### Glycerol Catalytic Conversion According to the invention

The reaction is carried out as in example 1, using a catalyst CuO/ZnO/Al₇O₃ (Alfa-Aesar HiFUEL™ R120) and a weight ratio of catalyst to glycerol was 0,4.

**Table 5**

| Glycerol conversion, % | Lower alcohol selectivity, % | 1,2-propanediol selectivity, % | 1,2-propanediol yield, % |
|---|---|---|---|
| 70,5 | 26,4 | 23,3 | 16,4 |

### Example 6 according to the invention

The reaction is carried out as in example 5 but with different methanol concentration.

**Table 6**

| Methanol concentration, w/w% | Glycerol conversion, % | Lower alcohol selectivity, % | 1,2-propanediol selectivity, % | 1,2-propanediol yield, % |
|---|---|---|---|---|
| 7,2 | 70,5 | 26,4 | 23,3 | 16,4 |
| 13,4 | 79,4 | 42 | 37,2 | 29,6 |

### Example 7 according to the invention

The reaction is carried out as in example 5 but without pre-reduction of the catalyst and for 24h reaction time.

**Table 7**

| Glycerol conversion, % | Lower alcohol selectivity, % | 1,2-propanediol selectivity, % | 1,2-propanediol yield, % |
|---|---|---|---|
| 59,3 | 15,5 | 9,7 | 5,8 |

### Example 8 according to the invention

The reaction is carried out as in example 5 but with different weight ratios of catalyst to glycerol.

**Table 8**

| Weight ratio catalyst/giycerol | Glycerol conversion, % | Lower alcohol selectivity, % | 1,2-propanediol selectivity, % | 1,2-propanediol yield, % |
|---|---|---|---|---|
| 0,2 | 44,8 | 44,3 | 40,8 | 18,3 |
| 0,4 | 70,5 | 26,4 | 23,3 | 16,4 |

### Example 9 according to the invention

### Glycerol Catalytic Conversion According to the invention

The reaction is carried out as in example 1, using synthesized Cu/Zn/Al catalyst by the co-precipitation method of different copper content and a weight ratio of catalyst to glycerol was 0,35.

### Catalyst: C: Cu/Zn/Al (1:1:1 atomic ratio), D: Cu/Zn/Al (6:3:1 atomic ratio)

**Table 9**

| Catalyst | Glycerol conversion, % | Lower alcohol selectivity, % | 1,2-propanediol selectivity, % | 1,2-propanediol yield, % |
|---|---|---|---|---|
| C | 82,7 | 32,5 | 30,9 | 25,5 |
| D | 84 | 37,5 | 34,7 | 29,2 |

### Example 10 according to the invention

### Glycerol Catalytic Conversion According to the invention

The reaction is carried out as in example 1, using synthesized Cu/Zn/Al by the gel-co precipitation of oxalate precursor method of different copper content and a weight ratio of catalyst to glycerol was 0,35.

### Catalyst: E: Cu/Zn/Al (1:1:1 atomic ratio), F: Cu/Zn/Al (6:3:1 atomic ratio)

**Table 10**

| Catalyst | Glycerol conversion, % | Lower alcohol selectivity, % | 1,2-propanediol selectivity, % | 1,2-propanediol yield, % |
|---|---|---|---|---|
| E | 76,3 | 33,1 | 28,4 | 21,7 |
| F | 88,8 | 47,3 | 39,2 | 34,8 |

## Claims

1. A catalytic process for hydrogenating glycerol for the production of lower alcohols, **characterized in that**
• first, a crude glycerol stream containing methanol mainly consisting of a mixture of glycerol, methanol and water is obtained in a process for producing biodiesel from transesterification of vegetable oils and fats,
• followed by a one-step catalytic process of glycerol conversion, wherein the crude glycerol stream is used avoiding separation steps wherein said water, methanol and glycerol reaction mixture is subjected to heating under inert nitrogen system pressure at temperatures of 220-260°C and a system initial pressure of 15-50 bar,
wherein the one-step catalytic process is independent of the addition of hydrogen gas or does not require the external addition of hydrogen gas, f
and wherein in the one-step catalytic process the reaction is carried out over a supported platinum metal catalyst or a bulk catalyst comprising copper.

2. The catalytic process according to the previous claims, **characterized in that** it is carried out over a supported metal catalyst, wherein the selected metal is supported on oxide-type supports, more particularly wherein the oxide-type supports are silicon oxide and aluminum oxide, and wherein the supported metal catalyst is Pt.

3. The catalytic process according to the preceding claim, wherein the carrier is loaded with metal between 1-8 wt%, more particularly wherein the carrier is loaded with metal between 4-6 wt%.

4. The catalytic process according to claim 2 or 3, **characterized in that** the supported metal catalyst is prepared by impregnation method.

5. The catalytic process according to any of the previous claims, **characterized in that** it is carried out over a bulk catalyst, comprising a first component metal which is Cu, particularly wherein the bulk catalyst further comprises one or two further components other than copper or copper oxide, more particularly a second component that is preferably zinc oxide, yet more particularly wherein a third component is a high surface area oxidic component, still more particularly wherein the third component is aluminum oxide.

6. The catalytic process according to the previous claim, wherein the weight ratio of the first component to the second component is between the range of 20/80 to 80/20, particularly wherein the weight ratio of the first component to the second component is between the range of 35/65 to 65/35, and/or wherein the third component content is 25 wt% or less.

7. The catalytic process according to claim 5 or 6, wherein the bulk catalyst is prepared by co-precipitation or gel-co precipitation of oxalate precursor techniques, particularly wherein the bulk catalyst is preferably prepared by gel-co precipitation of oxalate precursor technique.

8. The catalytic process according to any one of the preceding claims 1 to 7, wherein the concentration of glycerol is from 1-50 wt%, particularly from 5-30 wt% and preferably from 5-15 wt%, and/or wherein the concentration of methanol is from 1-30 wt%, preferably from 2-15 wt%, and/or wherein the concentration of water is from 40-94 wt%, preferably from 70-93 wt%.

9. The catalytic process according to any of the previous claims, wherein a glycerol conversion of 85% or higher is obtained, with a combined selectivity of lower alcohols of 45% or higher, particularly of which at least 39% is 1,2-propanediol in the former case of 45%.

10. The catalytic process according to any of the previous claims, wherein a 1,2-propanediol yield is 30% or higher.

11. The catalytic process according to any of the previous claims, wherein the main lower alcohols are from the group consisting of 1,2-propanediol, 1-propanol, 2-propanol, and/or whereby the process is to produce 1,2-propanediol.

12. The catalytic process according to any one of the preceding claims, wherein for the selective production of lower alcohols from crude glycerol stream consisting of glycerol, methanol and water, said process comprises a one-step in which a metal heterogeneous catalyst is used without external addition of hydrogen gas under inert nitrogen system pressure, wherein the hydrogen is produced in situ from methanol and water components and subsequently consumed by glycerol to form 1,2-propanediol.

13. The catalytic process according to any one of the preceding claims, wherein for converting the byproduct stream of biodiesel production into valuable chemical products, the process consists of a method for hydrogenating glycerol leading to the production of chemical 1,2-propanediol, without external hydrogen source, particularly wherein hydrogen is formed by the reaction of methanol and water, which constitutes the crude glycerol stream, and in situ consumed by glycerol to form 1,2-propylene glycol, more particularly wherein both the formation of hydrogen and its subsequent consumption by glycerol to form 1,2-propanediol are carried out by using the same apparatus, conditions and catalyst, more specifically wherein the catalyst comprises either a supported platinum, or a bulk catalyst consisting of copper and two oxidic components, such as zinc oxide and aluminum oxide.

## Patentansprüche

1. Katalytisches Verfahren zum Hydrieren von Glycerin für die Herstellung von niederen Alkoholen, **dadurch gekennzeichnet, dass**
• zuerst ein Strom von rohem Glycerin, das Methanol enthält, das hauptsächlich aus einer Mischung von Glycerin, Methanol und Wasser besteht, durch ein Verfahren zum Herstellen von Biodiesel aus der Umesterung von Pflanzenölen und -fetten erhalten wird,
• gefolgt von einem katalytischen Einschrittverfahren zur Glycerinumwandlung, wobei der Strom von rohem Glycerin unter Vermeidung von Trennschritten verwendet wird, wobei die Reaktionsmischung von Wasser, Methanol und Glycerin Erhitzen unter dem Druck eines inerten Stickstoffsystems bei Temperaturen von 220-260 °C und einem anfänglichen Systemdruck von 15-50 bar unterworfen wird,
wobei das katalytische Einschrittverfahren unabhängig von der Zugabe von Wasserstoffgas ist oder die externe Zugabe von Wasserstoffgas nicht erfordert,
und wobei bei dem katalytischen Einschrittverfahren die Reaktion über einem geträgerten Platinmetallkatalysator oder einem Massekatalysator, der Kupfer umfasst, ausgeführt wird.

2. Katalytisches Verfahren nach dem vorhergehenden Anspruch, **dadurch gekennzeichnet, dass** es über einem geträgerten Metallkatalysator ausgeführt wird, wobei das ausgewählte Metall auf Trägern vom Oxidtyp geträgert ist, wobei noch spezifischer die Träger vom Oxidtyp Siliciumoxid und Aluminiumoxid sind und wobei der geträgerte Metallkatalysator Pt ist.

3. Katalytisches Verfahren nach dem vorhergehenden Anspruch, wobei der Träger mit Metall zwischen 1-8 Gew.-% beladen ist, wobei noch spezifischer der Träger mit Metall zwischen 4-6 Gew.-% wobei beladen ist.

4. Katalytisches Verfahren nach Anspruch 2 oder 3, **dadurch gekennzeichnet, dass** der geträgerte Metallkatalysator durch ein Imprägnierungsverfahren hergestellt wird.

5. Katalytisches Verfahren nach einem der vorhergehenden Ansprüche, **dadurch gekennzeichnet, dass** es über einem Massekatalysator ausgeführt wird, der ein erstes Komponentenmetall umfasst, das Cu ist, wobei insbesondere der Massekatalysator ferner eine oder zwei weitere Komponenten außer Kupfer oder Kupferoxid, noch spezifischer eine zweite Komponente umfasst, die bevorzugt Zinkoxid ist, wobei selbst noch spezifischer eine dritte Komponente eine oxidische Komponente von hohem Oberflächenbereich ist, wobei sogar noch spezifischer die dritte Komponente Aluminiumoxid ist.

6. Katalytisches Verfahren nach dem vorhergehenden Anspruch, wobei das Gewichtsverhältnis der ersten Komponente zu der zweiten Komponente zwischen dem Bereich von 20/80 bis 80/20 liegt, wobei insbesondere das Gewichtsverhältnis der ersten Komponente zu der zweiten Komponente zwischen dem Bereich von 35/65 bis 65/35 liegt und/oder wobei der Gehalt der dritten Komponente 25 Gew.-% oder weniger beträgt.

7. Katalytisches Verfahren nach Anspruch 5 oder 6, wobei der Massekatalysator durch Copräzipitation oder Gelcopräzipitation von Oxalatvorläufertechniken hergestellt wird, wobei der Massekatalysator insbesondere bevorzugt durch Gelcopräzipitation von Oxalatvoräufertechnik hergestellt wird.

8. Katalytisches Verfahren nach einem der vorhergehenden Ansprüche 1 bis 7, wobei die Konzentration von Glycerin 1-50 Gew.-%, insbesondere 5-30 Gew.-% und bevorzugt 5-15 Gew.-% beträgt und/oder wobei die Konzentration von Methanol 1-30 Gew.-%, bevorzugt 2-15 Gew.-% beträgt und/oder wobei die Konzentration von Wasser 40-94 Gew.-%, bevorzugt 70-93 Gew.-% beträgt.

9. Katalytisches Verfahren nach einem der vorhergehenden Ansprüche, wobei eine Glycerinumwandlung von 85 % oder höher mit einer kombinierten Selektivität von niederen Alkoholen von 45 % oder höher erhalten wird, wovon insbesondere mindestens 39 % 1,2-Propandiol, in ersterem Falle 45 % sind.

10. Katalytisches Verfahren nach einem der vorhergehenden Ansprüche, wobei eine 1,2-Propandiolausbeute 30 % oder höher ist.

11. Katalytisches Verfahren nach einem der vorhergehenden Ansprüche, wobei die hauptsächlich niederen Alkohole aus der Gruppe stammen bestehend aus 1,2-Propandiol, 1-Propanol, 2-Propanol und/oder wobei das Verfahren dem Herstellen von 1,2-Propandiol dient.

12. Katalytisches Verfahren nach einem der vorhergehenden Ansprüche, wobei für die selektive Herstellung von niederen Alkoholen aus einem Strom von rohem Glycerin bestehend aus Glycerin, Methanol und Wasser, das Verfahren einen einzelnen Schritt umfasst, bei dem ein heterogener Metallkatalysator oder externen Zusatz von Wasserstoffgas unter Systemdruck von inertem Stickstoff verwendet wird, wobei der Wasserstoff in situ aus Methanol- uns Wasserkomponenten hergestellt und daraufhin durch Glycerin unter Bildung von 1,2-Propandiol aufgebraucht wird.

13. Katalytisches Verfahren nach einem der vorhergehenden Ansprüche, wobei zum Umwandeln des Nebenproduktstroms der Biodieselherstellung in wertvolle chemische Produkte das Verfahren aus einem Verfahren zum Hydrieren von Glycerin besteht, das zur Herstellung von chemischem 1,2 Propandiol ohne externe Wasserstoffquelle führt, wobei insbesondere Wasserstoff durch die Reaktion von Methanol und Wasser gebildet wird, was den Strom von rohem Glycerin darstellt, und in situ durch Glycerin aufgebraucht wird, um 1,2-Propylenglycol zu bilden, wobei noch spezifischer sowohl die Bildung von Wasserstoff und als auch sein darauffolgender Verbrauch durch Glycerin zum Bilden von 1,2 Propandiol durch Verwenden desselben Vorrichtung, Bedingungen und Katalysatoren ausgeführt werden, wobei noch spezifischer der Katalysator entweder ein geträgertes Platin oder einen Massekatalysator bestehend aus Kupfer und zwei oxidischen Komponenten wie Zinkoxid und Aluminiumoxid umfasst.

## Revendications

1. Procédé catalytique d'hydrogénation de glycérol pour la production d'alcools inférieurs, **caractérisé en ce que**
• tout d'abord, un flux de glycérol brut contenant du méthanol constitué principalement d'un mélange de glycérol, de méthanol et d'eau est obtenu suivant un procédé pour produire du biodiesel à partir de la transestérification d'huiles et de graisses végétales,
• suivi d'un processus catalytique en une étape de conversion du glycérol, dans lequel le flux de glycérol brut est utilisé en évitant les étapes de séparation dans lesquelles ledit mélange réactionnel eau, méthanol et glycérol est soumis à un chauffage sous pression d'un système d'azote inerte à des températures de 220-260°C et une pression initiale du système de 15-50 bars,
dans lequel le processus catalytique en une étape est indépendant de l'addition d'hydrogène gazeux ou ne nécessite pas l'addition externe d'hydrogène gazeux et dans lequel la réaction est effectuée, dans le processus catalytique en une étape, sur un catalyseur métallique au platine supporté ou un catalyseur en vrac comprenant du cuivre.

2. Procédé catalytique selon les revendications précédentes, **caractérisé en ce qu'**il est mis en œuvre sur un catalyseur métallique supporté, dans lequel le métal choisi est supporté sur des supports de type oxyde, plus particulièrement dans lequel les supports de type oxyde sont l'oxyde de silicium et l'oxyde d'aluminium et dans lequel le catalyseur métallique supporté choisi est le Pt.

3. Procédé catalytique selon la revendication précédente, dans lequel le support est chargé en métal entre 1-8% en poids, plus particulièrement dans lequel le support est chargé en métal entre 4-6% en poids.

4. Procédé catalytique selon la revendication 2 ou 3, **caractérisé en ce que** le catalyseur métallique supporté est préparé par un procédé d'imprégnation.

5. Procédé catalytique selon l'une quelconque des revendications précédentes, **caractérisé en ce qu'**il est mis en œuvre sur un catalyseur en vrac, comprenant un premier composant métallique, qui est le Cu, en particulier dans lequel le catalyseur en vrac comprend en outre un ou deux autres composants autres que le cuivre ou l'oxyde de cuivre, plus particulièrement un deuxième composant qui est de préférence de l'oxyde de zinc, encore plus particulièrement dans lequel un troisième composant est un composant oxydé à grande surface spécifique, toujours plus particulièrement dans lequel le troisième composant est de l'oxyde d'aluminium.

6. Procédé catalytique selon la revendication précédente, dans lequel le rapport pondéral du premier composant au deuxième composant est compris entre 20/80 et 80/20, en particulier dans lequel le rapport pondéral du premier composant au deuxième composant est compris dans la gamme allant de 35/65 à 65/35, et/ou dans lequel la teneur en troisième composant est de 25% en poids ou moins.

7. Procédé catalytique selon la revendication 5 ou 6, dans lequel le catalyseur en vrac est préparé par coprécipitation ou coprécipitation sur gel de techniques de précurseur d'oxalate, en particulier dans lequel le catalyseur en vrac est préparé de préférence par coprécipitation sur gel de la technique de précurseur d'oxalate.

8. Procédé catalytique selon l'une quelconque des revendications précédentes 1 à 7, dans lequel la concentration de glycérol est de 1-50% en poids, en particulier de 5-30% en poids et de préférence de 5-15% en poids, et/ou dans lequel la concentration de méthanol est de 1-30% en poids, de préférence de 2-15% en poids, et/ou dans lequel la concentration en eau est de 40-94% en poids, de préférence de 70-93% en poids.

9. Procédé catalytique selon l'une quelconque des revendications précédentes, dans lequel une conversion de glycérol de 85% ou plus est obtenue, avec une sélectivité combinée en alcools inférieurs de 45% ou plus, en particulier dont au moins 39% est 1,2-propanediol dans le premier cas de 45%.

10. Procédé catalytique selon l'une quelconque des revendications précédentes, dans lequel un rendement en 1,2-propanediol est de 30% ou plus.

11. Procédé catalytique selon l'une quelconque des revendications précédentes, dans lequel les alcools inférieurs principaux appartiennent au groupe constitué de 1,2-propanediol, 1-propanol, 2-propanol, et/ou dans lequel le procédé consiste à produire du 1,2-propanediol.

12. Procédé catalytique selon l'une quelconque des revendications précédentes, dans lequel pour la production sélective d'alcools inférieurs à partir d'un flux de glycérol brut constitué de glycérol, de méthanol et d'eau, ledit procédé comprend une étape dans laquelle un catalyseur hétérogène métallique est utilisé sans addition externe d'hydrogène gazeux sous la pression d'un système d'azote inerte, dans lequel l'hydrogène est produit in situ à partir de composants méthanol et eau et ensuite consommé par le glycérol pour former du 1,2-propanediol.

13. Procédé catalytique selon l'une quelconque des revendications précédentes, dans lequel pour convertir le flux de sous-produits de la production de biodiesel en produits chimiques de valeur, le procédé consiste en un procédé d'hydrogénation du glycérol conduisant à la production de 1,2-propanediol chimique, sans source externe d'hydrogène, en particulier dans lequel l'hydrogène est formé par la réaction du méthanol et de l'eau, qui constitue le flux de glycérol brut, et in situ consommé par le glycérol pour former du 1,2-propylène glycol, plus particulièrement dans lequel tant la formation d'hydrogène que sa consommation ultérieure par le glycérol pour former le 1,2-propanediol sont effectuées en utilisant le même appareil, les mêmes conditions et le même catalyseur, plus spécifiquement dans lequel le catalyseur comprend soit un platine supporté, soit un catalyseur en vrac constitué de cuivre et deux composants oxydiques, tels que l'oxyde de zinc et l'oxyde d'aluminium.
